# EUROPÄISCHE PATENTANMELDUNG

(11) **EP 3 029 017 A1**
(43) Veröffentlichungstag der Anmeldung: **08.06.2016**
(21) Anmeldenummer: 14196640.8
(22) Anmeldetag: 05.12.2014
(51) Int. Cl.: C07C 7/04, F25J 3/02, C07C 11/04

(54) **Verfahren und Anlage zur Herstellung von Kohlenwasserstoffen**

(71) Anmelder: Linde Aktiengesellschaft, 80331 München (DE)
(72) Erfinder: Fritz, Helmut, 81375 München (DE)
(74) Vertreter: Frommberger, Moritz

(57) **Zusammenfassung**

Es wird ein Verfahren zur Herstellung von Kohlenwasserstoffen, bei dem zumindest ein in einer Reaktionseinheit (1) erzeugter kohlenwasserstoffhaltiger Produktstrom (c) oder zumindest ein aus dem Produktstrom (c) gebildeter Strom (d, e, g, j, k, l) in zumindest einer Trenneinheit (10) unter Verwendung zumindest eines flüssigen, methanreichen Stroms (f, m) kryogen behandelt wird, vorgeschlagen. Dieses zeichnet sich dadurch aus, dass der zumindest eine methanreiche Strom (f, m) zumindest teilweise unter Verwendung eines druckbeaufschlagten, methanhaltigen Gasgemischs (A) erzeugt wird, das separat zu dem Produktstrom (c) bereitgestellt wird.

## Beschreibung

Die Erfindung betrifft ein Verfahren zur Herstellung von Kohlenwasserstoffen gemäß dem Oberbegriff des Patentanspruchs 1 und eine entsprechende Anlage.

### Stand der Technik

Verfahren zum Dampfspalten (engl. Steam Cracking) von Kohlenwasserstoffen sind bekannt und im Artikel "Ethylene" in Ullmann's Encyclopedia of Industrial Chemistry, Onlineausgabe, 15. April 2007, DOI 10.1002/14356007.a10_045.pub2, beschrieben.

Beim Dampfspalten, aber auch in anderen Verfahren, für die sich die vorliegende Erfindung ebenfalls eignet, werden Gemische von Kohlenwasserstoffen erhalten, die zumindest teilweise in die enthaltenen Komponenten aufgetrennt werden müssen. Dies kann mittels unterschiedlich ausgestalteter Trennsequenzen erfolgen, denen ein entsprechendes Kohlenwasserstoffgemisch beispielsweise nach einer sogenannten Rohgasverdichtung und weiteren Aufbereitungsschritten unterworfen wird.

Entsprechende Trennsequenzen für Dampfspaltverfahren sind ebenfalls aus dem Artikel "Ethylene" in Ullmann's Encyclopedia of Industrial Chemistry bekannt. Einer der Trennschritte in solchen Trennsequenzen dient zur Abtrennung von Methan und erfolgt in einem sogenannten Demethanizer, typischerweise unter Verwendung einer Destillationssäule. Am Kopf der Destillationssäule kann ein flüssiger, methanreicher Strom als Rücklauf aufgegeben werden. In entsprechenden Trennsequenzen können auch Absorptionskolonnen in Form sogenannter C2-Absorber zum Einsatz kommen, die ebenfalls mit einem flüssigen, methanreichen Strom als Rücklauf betrieben werden können. Auch an anderer Stelle entsprechender Trennsequenzen, beispielsweise in Wärmetauschern, kann ein flüssiger, methanreicher Strom als flüssiges Kältemittel mit deutlich unter -100 °C eingesetzt werden.

In derartigen Trennsequenzen wird also ein Produktstrom eines entsprechenden Verfahrens oder zumindest ein aus dem Produktstrom gebildeter Strom einer kryogenen Behandlung (Abkühlung in einem Wärmetauscher, Trennung in einer Destillationssäule und/oder Absorption in einer Absorptionskolonne) unterworfen, bei der zumindest ein flüssiger, methanreicher Strom zum Einsatz kommt. Der zumindest eine flüssige, methanreiche Strom wird in herkömmlichen Verfahren aus Methan des Produktstroms selbst gebildet.

Die Zusammensetzung der durch Dampfspaltverfahren erhaltenen Kohlenwasserstoffgemische wird maßgeblich über die Zusammensetzung der eingesetzten Kohlenwasserstoffgemische definiert. Je mehr Ethan letztere im Vergleich zu schwereren Einsätzen wie Naphtha enthalten, desto höher ist der Wasserstoffgehalt in den erhaltenen Kohlenwasserstoffgemischen. Der Gehalt an Methan, sowohl bezogen auf den Wasserstoffgehalt als auch bezogen auf den Gehalt an Ethan und Ethylen, verringert sich hingegen deutlich. Es kommt also selbst bei gleichbleibender Ethylenkapazität zu einer signifikanten Veränderung in der Zusammensetzung der erhaltenen Kohlenwasserstoffgemische, insbesondere zu einer Reduzierung der Methanproduktion. Dies kann insbesondere bei der Umrüstung oder teilweisen Umrüstung von Dampfspaltanlagen zu Problemen führen.

Aufgrund des "fehlenden" Methans, das in seiner Menge für die genannten Zwecke nicht mehr ausreicht und/oder aufgrund seines deutlich reduzierten Anteils (und damit Partialdrucks) nicht mehr zugänglich ist, d.h. verflüssigt werden kann, kann es ohne zusätzliche Maßnahmen bei einer entsprechenden Umrüstung dazu kommen, dass die Anlage nicht mehr in der Lage ist, die gewünschten Produktreinheiten und/oder Produktausbeuten zu liefern.

Jedoch kann es aus wirtschaftlichen Gründen wünschenswert sein, den Ethananteil in den in Dampfspaltverfahren eingesetzten Kohlenwasserstoffgemischen auch in bestehenden Anlagen zu erhöhen. Ethanreiche Kohlenwasserstoffgemische fallen bei der Erdgasproduktion in großen Mengen an, unter anderem in Form sogenannter Erdgaskondensate (engl. Natural Gas Liquids, NGL), und können durch Dampfspalten zu Wertprodukten umgesetzt werden. Entsprechendes gilt auch für das vergleichsweise ethanreiche Schiefergas (engl. Shale Gas).

Die genannten Probleme lassen sich entweder durch eine Erhöhung des Methananteils durch eine gezielte Rückführung von Methan oder durch die Bereitstellung eines separaten Kältekreislaufs, beispielsweise eines Methankreislaufs, adressieren.

In ersterem Fall könnte das nach seiner Verwendung als Kältemittel vorliegende Methan oder ein entsprechendes methanhaltiges Kohlenwasserstoffgemisch vor oder in die Rohgasverdichtung, beispielsweise vor einen Rohgasverdichter oder zwischen einzelne Verdichterstufen eines Rohgasverdichters, zurückgeführt und hier mit dem Gasgemisch aus dem Dampfspaltverfahren vereinigt werden. Das in jedem Zyklus stromab abgeschiedene und zurückgeführte Methan akkumuliert sich auf diese Weise, so dass sein Anteil und damit sein Partialdruck auch bei geringerer Neubildung für die genannten Zwecke ausreicht.

Bei einer derartigen Rückführung vor oder in die Rohgasverdichtung erhöht sich jedoch zwangsläufig das zu verdichtende und in den nachfolgenden Trennschritten zu bearbeitende Gasvolumen, wodurch aufwendige Erweiterungen erforderlich sind. (Wie erwähnt, verringert sich die Menge an Methan zugunsten einer erhöhten Wasserstoffbildung, so dass eine umgerüstete Anlage bereits hierdurch in ihrer Kapazität ausgelastet ist bzw. durch die reduzierte Methanbildung keine entsprechenden Kapazitäten frei werden.)

In einem separaten Methankreislauf müsste das nach der Verwendung als Kältemittel bei niedrigem Druck vorliegende Methan oder ein entsprechendes methanhaltiges Kohlenwasserstoffgemisch jeweils stark verdichtet (rückverdichtet) werden, um seine anschließende Verflüssigung ermöglichen zu können. In diesem Fall wären aufwendige separate Verdichter erforderlich.

Alternativ oder zusätzlich zu einem Methankreislauf kann auch eine anderweitige Bereitstellung von Kälteleistung auf hinreichend tiefem Temperaturniveau erfolgen, beispielsweise durch Entspannung von Wasserstoff. Auch in diesem Fall ergibt sich jedoch ein beträchtlicher Aufwand für die Bereitstellung von Kältemaschinen und/oder Expansionsturbinen.

Die erläuterten Maßnahmen zum Ausgleich einer verringerten Methanproduktion sind zwar grundsätzlich in bestehenden Anlagen nachrüstbar, aber mit entsprechend hohen Investitionskosten und/oder Eingriffen in eine Anlage verbunden. Die vorliegende Erfindung stellt sich daher die Aufgabe, die Trennung von Gasgemischen aus entsprechenden Verfahren zu verbessern.

### Offenbarung der Erfindung

Diese Aufgabe wird durch ein Verfahren und eine Anlage zur Herstellung von Kohlenwasserstoffen mit den Merkmalen der unabhängigen Patentansprüche gelöst. Bevorzugte Ausgestaltungen sind Gegenstand der abhängigen Patentansprüche und der nachfolgenden Beschreibung.

Vor der Erläuterung der Merkmale und Vorteile der vorliegenden Erfindung werden deren Grundlagen und die verwendeten Begriffe erläutert.

Flüssige und gasförmige Ströme können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9%, 99,99% oder 99,999% und "arm" für einen Gehalt von höchstens 50%, 25%, 10%, 5%, 1%, 0,1%, 0,01% oder 0,001% auf molarer, Gewichts- oder Volumenbasis stehen kann. Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält. Ist hier von "flüssigem Methan" die Rede, sei darunter ein Strom verstanden, der reich an Methan ist, jedoch nicht ausschließlich aus Methan bestehen muss.

Dampfspaltverfahren werden im kommerziellen Maßstab nahezu ausschließlich in Rohrreaktoren durchgeführt, in denen einzelne Reaktionsrohre (in Form von Rohrschlangen, sogenannten Coils) oder Gruppen von entsprechenden Reaktionsrohren auch bei unterschiedlichen Spaltbedingungen betrieben werden können. Unter gleichen oder vergleichbaren Spaltbedingungen betriebene Reaktionsrohre oder Gruppen von Reaktionsrohren, ggf. aber auch unter einheitlichen Spaltbedingungen betriebene Rohrreaktoren insgesamt, werden auch als "Spaltöfen" bezeichnet. Eine Anlage zum Dampfspalten (auch als "Olefinanlage" bezeichnet) kann einen oder mehrere Spaltöfen aufweisen. In der im Rahmen der vorliegenden Anmeldung beschriebenen Dampfspalteinheit können ein oder mehrere entsprechender Spaltöfen vorgesehen sein.

Einem Spaltofen wird ein sogenannter "Ofeneinsatz" zugeführt und in diesem zumindest teilweise umgesetzt. Als Ofeneinsatz eignet sich eine Vielzahl von Kohlenwasserstoffen und Kohlenwasserstoffgemischen von Ethan bis Gasöl bis zu einem Siedepunkt von typischerweise 600 °C. Ein Ofe neinsatz kann aus einem sogenannten "Frischeinsatz" bestehen, also aus einem Einsatz, der anlagenextern bereitgestellt und beispielsweise aus einer oder mehreren Erdölfraktionen, Erdgas und/oder Erdgaskondensaten gewonnen wird. Ein Ofeneinsatz kann auch aus einem oder mehreren sogenannten "Recycleströmen" bestehen, also Strömen, die in der Anlage selbst erzeugt und in einen entsprechenden Spaltofen zurückgeführt werden. Ein Ofeneinsatz kann auch aus einem Gemisch eines oder mehrerer Frischeinsätze mit einem oder mehreren Recycleströmen bestehen.

Einem oder mehreren Spaltöfen wird ein sogenanntes "Rohgas" entnommen und geeigneten Nachbehandlungsschritten unterworfen. Diese umfassen zunächst eine Aufbereitung des Rohgases, beispielsweise durch Quenchen, Kühlen und Trocknen, wodurch ein sogenanntes "Spaltgas" erhalten wird.

Gängige Verfahren zur Trennung von Produktströmen aus Dampfspaltverfahren oder anderen Kohlenwasserstoffgemischen umfassen insbesondere die Trennung in eine Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (jedoch konventionsgemäß unter Umständen auch Wasserstoff, dann auch "C1minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt für die "C5-Fraktion" und die höheren Fraktionen. Mehrere solcher Fraktionen können auch Verfahrens- und/oder bezeichnungsmäßig zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen.

Zur kryogenen Trennung können insbesondere die bereits erwähnten Destillationssäulen und Absorptionskolonnen zum Einsatz kommen. Zur Auslegung und Ausgestaltung entsprechender Vorrichtungen sei auf einschlägige Lehrbücher verwiesen (siehe beispielsweise K. Sattler: Thermische Trennverfahren. Grundlagen, Auslegung, Apparate. Weinheim: Wiley-VCH, 3. Auflage 2001). Derartige Trenneinheiten werden nachfolgend unter dem Begriff "Trennsäulen" zusammengefasst. Eine im Rahmen der vorliegenden Erfindung verwendete Trennsäule wird bei tiefkalten Temperaturen betrieben und ist zur Tieftemperatur-Gaszerlegung eingerichtet. Einer Trennsäule ist typischerweise immer zumindest eine flüssige Fraktion ("Sumpfprodukt") und eine gasförmige Fraktion ("Kopfprodukt") in einem oberen ("Kopf") bzw. unteren Bereich ("Sumpf") entnehmbar.

Bei einer "Destillationssäule" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, bereitgestelltes Stoffgemisch (Fluid) zumindest teilweise aufzutrennen, also aus dem Stoffgemisch jeweils Reinstoffe oder Stoffgemische zu erzeugen, die gegenüber dem Stoffgemisch bezüglich zumindest einer Komponente angereichert bzw. abgereichert oder reich bzw. arm im oben erläuterten Sinne sind. Destillationssäulen sind aus dem Bereich der Trenntechnik hinlänglich bekannt. Typischerweise sind Destillationssäulen als zylindrische Metallbehälter ausgebildet, die mit Einbauten, beispielsweise Siebböden oder geordneten oder ungeordneten Packungen, ausgerüstet sind. Eine Destillationssäule zeichnet sich unter anderem dadurch aus, dass sich in ihrem unteren Bereich, auch als Sumpf bezeichnet, eine flüssige Fraktion abscheidet. Diese flüssige Fraktion, die hier als Sumpfflüssigkeit bezeichnet wird, wird in einer Destillationssäule mittels eines Sumpfverdampfers erwärmt, so dass kontinuierlich ein Teil der Sumpfflüssigkeit verdampft und in der Destillationssäule gasförmig aufsteigt. Eine Destillationssäule ist ferner typischerweise mit einem sogenannten Kopfkondensator versehen, in den zumindest ein Teil eines sich in einem oberen Bereich der Destillationssäule anreichernden Gasgemischs oder ein entsprechendes Reingas, hier als Kopfgas bezeichnet, eingespeist, zu einem Teil zu einem Kondensat verflüssigt und als flüssiger Rücklauf am Kopf der Destillationssäule aufgegeben wird. Ein Teil des aus dem Kopfgas erhaltenen Kondensats kann anderweitig verwendet werden.

Im Gegensatz zu einer Destillationssäule verfügt eine "Absorptionskolonne" typischerweise nicht über einen Sumpfverdampfer. Auch Absorptionskolonnen sind aus dem Bereich der Trenntechnik allgemein bekannt. Absorptionskolonnen werden zur Absorption im Phasengegenstrom verwendet und daher auch als Gegenstromkolonnen bezeichnet. Bei der Absorption im Gegenstrom strömt die abgebende Gasphase aufwärts durch eine Absorptionskolonne. Die aufnehmende Lösungsphase fließt, von oben aufgegeben und unten abgezogen, der Gasphase entgegen. Die Gasphase wird mit der Lösungsphase "gewaschen". In einer entsprechenden Absorptionskolonne sind ebenfalls typischerweise Einbauten vorgesehen, die für einen stufenweisen (Böden, Sprühzonen, rotierende Teller usw.) oder stetigen (regellose Schüttungen von Füllkörpern, Packungen usw.) Phasenkontakt sorgen. Am Kopf einer solchen Absorptionskolonne wird ein gasförmiges Fluid erhalten, das als "Kopfprodukt" aus dieser abgezogen werden kann. Im Sumpf der Absorptionskolonne scheidet sich eine Flüssigkeit ab, die als "Sumpfprodukt" abgezogen werden kann. Die Gasphase wird in der Absorptionskolonne bezüglich einer oder mehrerer Komponenten abgereichert, welche in das Sumpfprodukt übergehen.

### Vorteile der Erfindung

Ein wesentlicher Aspekt der vorliegenden Erfindung ist es, einen flüssigen, methanreichen Strom nicht anlagenintern zu bilden, sondern von extern in der benötigten Zusammensetzung genau dorthin zu bringen, wo er für eine entsprechende kryogene Behandlung erforderlich ist, insbesondere in einen Demethanizer. Ein "Demethanizer" umfasst dabei gemäß dem hier verwendeten Sprachgebrauch nicht nur die entsprechende Destillationssäule selbst, sondern auch die zugeordneten Einrichtungen und Apparate wie Abscheidbehälter, Wärmetauscher, Absorptionskolonnen und dergleichen.

Die Erfindung schlägt hierzu ein Verfahren zur Herstellung von Kohlenwasserstoffen vor, bei dem zumindest ein in einer Reaktionseinheit erzeugter kohlenwasserstoffhaltiger Produktstrom oder zumindest ein aus dem Produktstrom gebildeter Strom in zumindest einer Trenneinheit unter Verwendung zumindest eines flüssigen, methanreichen Stroms kryogen behandelt wird.

Wie zuvor erläutert, umfasst eine "kryogene Behandlung" im hier verwendeten Sprachgebrauch beispielsweise eine Abkühlung in einem Wärmetauscher, eine Trennung in einer Destillationssäule und/oder eine Absorption in einer Absorptionskolonne, bei der zumindest ein flüssiger, methanreicher Strom zum Einsatz kommt. Der zumindest eine flüssige, methanreiche Strom wird in herkömmlichen Verfahren, wie erwähnt, aus Methan des Produktstroms selbst gebildet.

Das erfindungsgemäße Verfahren sieht vor, dass der zumindest eine methanreiche Strom zumindest teilweise unter Verwendung eines druckbeaufschlagten, methanhaltigen Gasgemischs erzeugt wird, das separat zu dem Produktstrom bereitgestellt wird. Mit anderen Worten wird im Rahmen der vorliegenden Erfindung der flüssige, methanreiche Strom nicht aus dem Produktstrom selbst gebildet, wie beispielsweise in herkömmlichen Trennsequenzen zur Trennung von Gasgemischen aus Dampfspaltverfahren, es wird hingegen vielmehr ein bereits druckbeaufschlagtes Gasgemisch extern zugeführt.

Durch die erfindungsgemäß vorgeschlagenen Maßnahmen werden die zuvor erläuterten Nachteile überwunden. So ist insbesondere keine Implementierung eines Methankreislaufs oder einer Methanrückführung erforderlich. Wird im Rahmen der vorliegenden Erfindung eine Quelle für ein druckbeaufschlagtes, methanhaltiges Gasgemisch eingesetzt, in der dieses bereits auf einen geeigneten Druck verdichtet ist, sind keine zusätzlichen Verdichter mehr erforderlich, um den flüssigen, methanreichen Strom bereitstellen zu können. Allenfalls muss ein entsprechendes Gasgemisch entspannt werden, wodurch Kälte und/oder Wellenleistung erzeugt werden kann. Derartige vorteilhafte Drücke liegen in Erdgaspipelines vor.

Die Erfindung entfaltet besondere Vorteile bei den eingangs erwähnten Destillationssäulen und Absorptionskolonnen. Durch die Verwendung des flüssigen, methanreichen Rücklaufs, der aufgrund seiner Herkunft im Wesentlichen frei von Ethylen ist, ermöglicht die vorliegende Erfindung eine vorteilhafte Gewinnung von im Wesentlichen ethylenfreien Fraktionen. Der Verlust an Ethylen wird damit durch den Einsatz der vorliegenden Erfindung minimiert.

Die vorliegende Erfindung kann insbesondere in Verfahren zum Dampfspalten eingesetzt werden, bei denen die Reaktionseinheit als Dampfspalteinheit mit wenigstens einem Spaltofen ausgebildet ist. Die vorliegende Erfindung eignet sich hier insbesondere zur Umrüstung (Revamp) von bestehenden, zum Dampfspalten von flüssigen Kohlenwasserstoffeinsätzen eingerichteten Dampfspaltanlagen und zum Ausgleich einer umrüstungsbedingten Verringerung der Methanproduktion, wie eingangs erläutert. Die vorliegende Erfindung erlaubt eine Umrüstung entsprechender Anlagen lediglich durch Bereitstellen eines Aufbereitungsabschnitts für das druckbeaufschlagte, methanhaltige Gasgemisch, wie unten erläutert.

Für die genannten Zwecke, also ein Abkühlen in zumindest einem mit dem zumindest einen methanreichen Strom als Kältemittel betriebenen Wärmetauscher und/oder ein Trennen in zumindest einer kryogenen Trenneinrichtung (Destillationssäule und/oder Absorptionskolonne), in der der zumindest eine methanreiche Strom als Rücklauf aufgegeben wird, muss Methan verflüssigt sein bzw. werden, weshalb ein entsprechender Mindestdruck erforderlich ist.

Diese Anforderung erfüllt insbesondere druckbeaufschlagtes Erdgas aus einer entsprechenden Pipeline. Derart bereitgestelltes Erdgas weist bereits den erforderlichen Druck auf, der es ermöglicht, eine Verflüssigung zu erzielen und damit einen flüssigen Rücklauf oder einen entsprechenden Strom für einen Wärmetauscher bereitzustellen. Beispielsweise liegt Erdgas in entsprechenden Pipelines bei 40 bis 60 bar vor, der Druck liegt also deutlich oberhalb des für die Verflüssigung erforderlichen Mindestdrucks von mindestens 28 bar abs. (bei ca. -97 °C). Zur üblichen Verwendung als Brenngas erfolgt herkömmlicherweise eine Entspannung auf einen Druck von beispielsweise weniger als 9 bar abs. Erfindungsgemäß wird hingegen entsprechend verdichtetes Erdgas auf höhere Drücke, beispielsweise ca. 30 bis 40 bar abs., entspannt oder auf einem entsprechend hohen Druck eingesetzt.

Vor der Verwendung wird ein entsprechendes druckbeaufschlagtes, methanhaltiges Gasgemisch, beispielsweise Erdgas, vorteilhafterweise von störenden Komponenten befreit, sofern es diesbezüglich nicht bereits die jeweiligen Anforderungen erfüllt. Die Frage, welche Komponenten als "störend" anzusehen sind, richtet sich nach der angestrebten Verwendung, also der kryogenen Behandlung, die mit dem wenigstens einen flüssigen, methanreichen Strom durchgeführt werden soll. Bei einer reinen Verwendung als Kältemittel sind keine besonderen Reinheitserfordernisse zu beachten. Ein entsprechendes druckbeaufschlagtes, methanhaltiges Gasgemisch muss hier nur frei von Wasser und Kohlendioxid sowie ggf. von korrosiven Bestandteilen sein. Bei der Verwendung in einer Destillationssäule oder Absorptionskolonne ergeben sich hingegen höhere Anforderungen, die sich insbesondere nach der konkret realisierten Trennsequenz richten:
Handelt es sich bei der Abtrennung von Methan im Demethanizer um den ersten Trennschritt (in sogenannten "Frontend Demethanizer"- oder "Demethanizer First"-Verfahren), sind hier auch noch Kohlenwasserstoffe mit zwei, drei und mehr Kohlenstoffatomen in dem Gemisch enthalten. In diesem Fall kann ein gewisser Gehalt an Kohlenwasserstoffen mit drei und mehr Kohlenstoffatomen in dem flüssigen, methanreichen Strom toleriert werden, sofern diese die Kapazität nachgeschalteter Trenneinrichtungen nicht überlasten.

Kommt stromauf ein sogenannter Deethanizer zum Einsatz (in sogenannten "Frontend Deethanizer"- oder "Deethanizer First"-Verfahren), weist das Gasgemisch noch Kohlenwasserstoffe mit zwei Kohlenstoffatomen, aber keine nennenswerten Mengen von Kohlenwasserstoffen mit drei und mehr Kohlenstoffatomen mehr auf. In diesem Fall ist daher zu vermeiden, mit dem flüssigen, methanreichen Strom erneut Kohlenwasserstoffe mit drei und mehr Kohlenstoffatomen einzubringen. Kohlenwasserstoffe mit zwei Kohlenstoffatomen können hingegen auch hier in gewissem Umfang toleriert werden.

Wurden stromauf nur Kohlenwasserstoffe mit vier und mehr Kohlenstoffatomen abgetrennt (in sogenannten "Frontend Depropanizer"- oder "Depropanizer First"-Verfahren), sind im Gasgemisch im Wesentlichen noch Kohlenwasserstoffe mit drei Kohlenstoffatomen enthalten. In diesem Fall kann daher ein gewisser Gehalt an Kohlenwasserstoffen mit drei (aber nicht mehr) Kohlenstoffatomen in dem flüssigen, methanreichen Strom toleriert werden.

Vorteilhafterweise wird der flüssige, methanreiche Strom zumindest teilweise aus einem flüssigen Strom erzeugt, welcher wiederum unter Verwendung eines geeigneten Destillations- bzw. Rektifikationsverfahrens aus dem druckbeaufschlagten, methanhaltigen Gasgemisch, beispielsweise dem Erdgas, gebildet wird. Der flüssige, methanreiche Strom kann in dieser Weise gezielt von den erwähnten störenden Komponenten befreit werden, wobei sich die Ausgestaltung eines entsprechenden destillativen Verfahrens nach der zu erzielenden Reinheit richten kann. In bestimmten Fällen kann auf ein destillatives Verfahren verzichtet werden. Je nach der erforderlichen Aufreinigung ist eine entsprechende Vorbehandlung unter Verwendung von Verdichtern, Pumpen, Adsorbern etc. vorzusehen, ehe ggf. ein Destillations- bzw. Rektifikationsverfahren durchgeführt wird.

Vorteilhafterweise wird das druckbeaufschlagte, methanhaltige Gasgemisch zunächst zumindest teilweise unter Druck von Verunreinigungen befreit. Die Aufreinigung unter Druck ist besonders vorteilhaft, weil entsprechende Reinigungseinrichtungen aufgrund der Verdichtung nur zur Behandlung vergleichsweise geringer Volumenströme ausgebildet sein müssen.

Wasser und Kohlendioxid sind dabei auf jeden Fall aus dem methanhaltigen Gasgemisch zu entfernen, um ein Ausfrieren bei und nach der anschließenden Abkühlung zu vermeiden. Die weiteren zu entfernenden Verunreinigungen richten sich nach der späteren Verwendung des flüssigen, methanreichen Stroms:
Vorteilhafterweise werden zur Befreiung von entsprechenden Verunreinigungen beispielsweise Arsin, Phosphin, Schwefelverbindungen, Kohlendioxid und/oder Quecksilber zumindest teilweise adsorptiv aus dem druckbeaufschlagten, methanhaltigen Gasgemisch entfernt. Zur Regeneration der dabei verwendeten Adsorber kann auch ein in einem Destillations- bzw. Rektifikationsverfahren anfallender Strom, beispielsweise ein verflüssigtes Kopfgas (z.B. stickstoffhaltiges Kopfgas aus einer Trennsäule, die zur Bereitstellung des flüssigen, methanreichen Stroms verwendet wird) oder ein wiederverdampftes Sumpfprodukt verwendet werden.

Besonders vorteilhaft ist es, wenn das druckbeaufschlagte, methanhaltige Gasgemisch in dem Destillationsverfahren an Stickstoff, Wasserstoff und/oder Helium abgereichert wird. Die Entfernung derartiger Komponenten ist insbesondere deshalb vorteilhaft, weil diese möglicherweise in die Produkte übergehen könnten.

Besonders vorteilhaft ist hierbei die Verwendung einer Trennwandkolonne. In eine derartige Trennwandkolonne kann auf einer Seite der Trennwand ein Methan, Stickstoff, Wasserstoff und/oder Helium sowie höhere Kohlenwasserstoffe enthaltendes Gasgemisch eingespeist werden, auf der anderen Seite der Trennwand wird hingegen ein an höheren Kohlenwasserstoffen sowie Stickstoff, Wasserstoff und/oder Helium abgereichertes, flüssiges Gasgemisch erhalten.

Die Erfindung schlägt ferner eine Anlage vor, die zur Durchführung eines Verfahrens eingerichtet ist, wie es zuvor erläutert wurde, und die sämtliche, zur Durchführung eines entsprechenden Verfahrens eingerichtete Mittel aufweist. Insbesondere weist eine derartige Anlage Mittel zur Bereitstellung des flüssigen, methanreichen Stroms und zu dessen Erzeugung aus einem druckbeaufschlagten, methanhaltigen Gasgemisch auf. Diese Mittel umfassen insbesondere eine Destillationssäule, beispielsweise eine Trennwandkolonne.

Die Erfindung wird unter Bezugnahme auf die beigefügten Zeichnungen näher erläutert, welche bevorzugte Ausführungsformen der Erfindung zeigen.

### Kurze Beschreibung der Zeichnungen

Figur 1 zeigt eine Anlage zur Herstellung von Kohlenwasserstoffen gemäß dem Stand der Technik in vereinfachter schematischer Darstellung.
Figur 2 zeigt eine Anlage zur Herstellung von Kohlenwasserstoffen gemäß dem Stand der Technik in vereinfachter schematischer Darstellung.
Figur 3 zeigt eine Anlage zur Herstellung von Kohlenwasserstoffen gemäß einer Ausführungsform der Erfindung in vereinfachter schematischer Teildarstellung.
Figur 4 zeigt eine Anlage zur Herstellung von Kohlenwasserstoffen gemäß einer Ausführungsform der Erfindung in vereinfachter schematischer Teildarstellung.
Figur 5 zeigt eine Anlage zur Herstellung von Kohlenwasserstoffen gemäß einer Ausführungsform der Erfindung in vereinfachter schematischer Teildarstellung.

In den Figuren sind einander entsprechende Elemente mit identischen Bezugszeichen veranschaulicht. Auf eine wiederholte Erläuterung entsprechender Elemente wird der Übersichtlichkeit halber verzichtet.

### Ausführliche Beschreibung der Zeichnungen

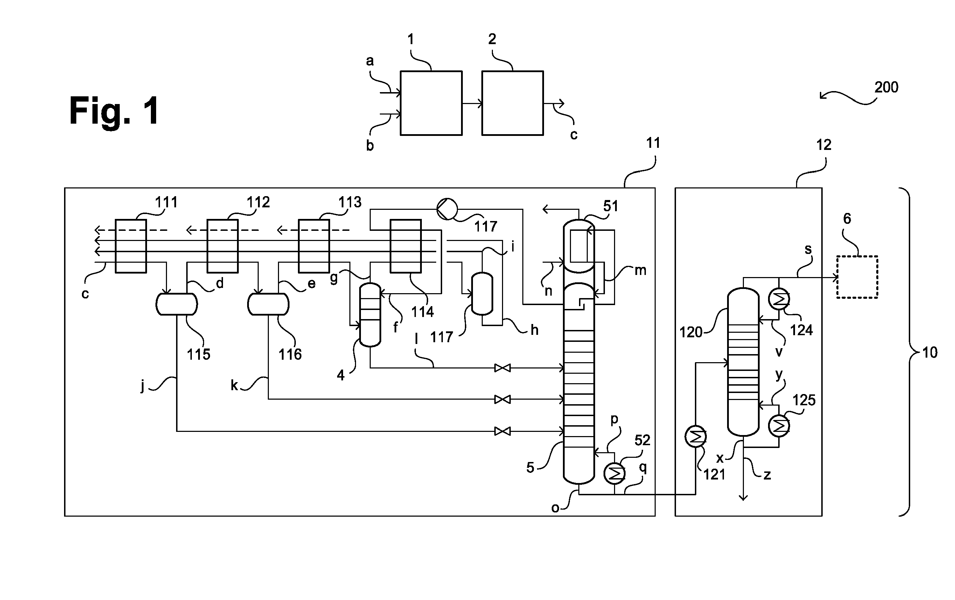

Figur 1 veranschaulicht eine Anlage zur Herstellung von Kohlenwasserstoffen gemäß dem Stand der Technik. Die Anlage ist insgesamt mit 200 bezeichnet.

Die Anlage 200 umfasst im dargestellten Beispiel eine als Dampfspalteinheit 1 ausgebildete Reaktionseinheit, beispielsweise einen oder mehrere in an sich bekannter Weise ausgebildete Spaltöfen wie oben erläutert. Der Dampfspalteinheit 1 werden im dargestellten Beispiel zwei Einsatzströme a, b zugeführt, die beispielsweise sogenannten Frischeinsatz, also von der Anlagengrenze der Anlage 100 zugeführte kohlenwasserstoffhaltige Gas- oder Flüssigkeitsgemische, jedoch auch sogenannte Recycleströme, also in der Anlage 200 selbst erzeugte kohlenwasserstoffhaltige Gasgemische, zugeführt werden können. Mehrere Einsatzströme a, b können auf mehrere, bei gleichen oder unterschiedlichen Spaltbedingungen betriebene Spaltöfen verteilt und/oder separaten, bei gleichen oder unterschiedlichen Spaltbedingungen betriebenen Spaltöfen zugeführt werden.

Der Dampfspalteinheit 1 kann insgesamt ein kohlenwasserstoffhaltiger Produktstrom c entnommen werden. Der Produktstrom c kann aus einem oder mehreren Spaltöfen der Dampfspalteinheit 1 stammen. Der Produktstrom c enthält neben Kohlenwasserstoffen, die in der Dampfspalteinheit 1 aus eingesetzten Kohlenwasserstoffen der Einsatzströme a, b erzeugt werden, auch nicht umgesetzte Verbindungen der Einsatzströme a, b. Wie erläutert setzt sich ein Produktstrom eines in einer entsprechenden Dampfspalteinheit 1 implementierten Dampfspaltverfahrens typischerweise aus Wasserstoff sowie Kohlenwasserstoffen mit einem (Methan) bis über 20 Kohlenstoffatomen zusammen. Wie ebenfalls erläutert, richtet sich die Verteilung der jeweiligen Kettenlängen dabei unter anderem nach der Zusammensetzung der Einsatzströme a, b.

Der Produktstrom c entstammt mehreren, hier nicht im Detail gezeigten Schritten 2 einer sogenannten Rohgasaufbereitung, beispielsweise einem Ölquench und einer Wasserwäsche. Nach einer nicht gezeigten Rohgasverdichtung und ggf. vorgesehenen, sogenannten Frontend-Hydrierungsschritten wird der Produktstrom c anschließend einer Trenneinheit 10 zugeführt, die im unteren Teil der Figur 1 ausschnittsweise veranschaulicht ist.

Die Trenneinheit 10 der Anlage 200 implementiert ein "Demethzanizer First"-Verfahren, d.h. der Produktstrom c wird zunächst einem Demethanizer zugeführt, der hier insgesamt mit 11 bezeichnet ist. Dem Demethanizer 11 ist ein hier insgesamt mit 12 bezeichneter Deethanizer 12 nachgeschaltet. Bei dem Strom c handelt es sich um einen gasförmigen, auf beispielsweise 36 bar abs. verdichteten und abgekühlten Kohlenwasserstoffstrom, der Wasserstoff, Methan und Kohlenwasserstoffe mit zwei und mehr Kohlenstoffatomen enthält. Methan und Wasserstoff werden in dem Demethanizer 11 abgetrennt.

Der Demethanizer 11 ist stark vereinfacht dargestellt. Auf Die Darstellung einer Vielzahl von Strömen, Ventilen, Wärmetauschern, Behältern usw. wurde verzichtet. Der Demethanizer 11 umfasst im Beispiel vier (Gegenstrom-)Wärmetauscher 111 bis 114, kann jedoch auch mehr oder weniger entsprechender Wärmetauscher und weitere Wärmetauscher aufweisen. Die Wärmetauscher 111 bis 114 können neben den nachfolgend erläuterten Strömen insbesondere mit geeigneten Kältemittelströmen, hier gestrichelt gezeigt, gekühlt werden.

Der Strom c wird zunächst durch den Wärmetauscher 111 geführt, abgekühlt und anschließend in einen Flüssigkeitsabscheider 115 eingespeist. Hier gasförmig verbleibendes Fluid wird als Strom d durch den Wärmetauscher 112 geführt, abgekühlt und in einen Flüssigkeitsabscheider 116 eingespeist. Auch hier gasförmig verbleibendes Fluid wird als Strom e durch den Wärmetauscher 113 geführt, weiter abgekühlt und bei beispielsweise ca. 35 bar abs. und ca. -100 °C in eine Absorptionskolonne 4, den mehrfach erwähnten C2-Absorber, überführt.

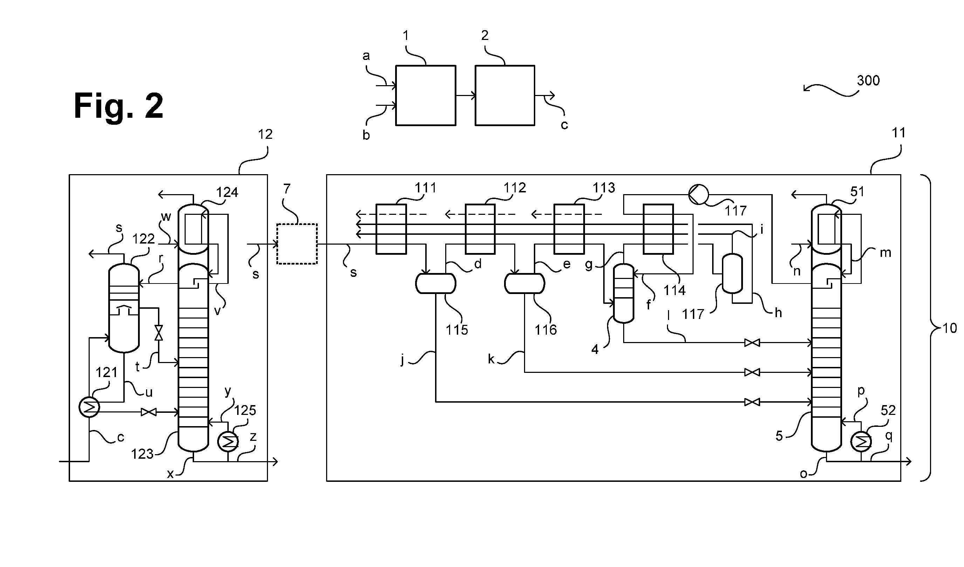

Am Kopf der Absorptionskolonne 4 wird ein flüssiger methanreicher Strom f aufgegeben, der in dem Strom e noch enthaltene Kohlenwasserstoffe mit zwei und mehr Kohlenstoffatomen aus diesem auswäscht. Der flüssige methanreiche Strom f könnte auch noch gewisse Anteile an Kohlenwasserstoffen mit zwei Kohlenstoffatomen enthalten, da diese in den Sumpf der Absorptionskolonne 4 übergehen. Im dargestellten Beispiel eines "Demethanizer First"-Verfahrens kann der flüssige methanreiche Strom f ferner auch noch gewisse Anteile an Kohlenwasserstoffen mit drei und mehr Kohlenstoffatomen enthalten, da auch diese in den Sumpf der Absorptionskolonne 4 übergehen und in dem nachgeschalteten Deethanizerschritt abgetrennt werden können. In einem "Deethanizer First"-Verfahren, wie es in Figur 2 veranschaulicht ist, wäre letzteres hingegen zu vermeiden. Im Sumpf der Absorptionskolonne 4 fällt damit ein Gemisch aus Methan und entsprechenden Kohlenwasserstoffen an, am Kopf der Absorptionskolonne 4 hingegen ein Gemisch, das im Wesentlichen aus Methan und Wasserstoff besteht und vorzugsweise frei von Kohlenwasserstoffen mit zwei und mehr Kohlenstoffatomen ist.

Letzteres wird als Strom g vom Kopf der Absorptionskolonne 4 abgezogen, in dem Gegenstromwärmetauscher 114 auf eine Temperatur unterhalb der Siedetemperatur von Methan bei dem genannten Druck abgekühlt und anschließend in einen Wasserstoffabscheider 117 überführt. Aus dem Wasserstoffabscheider 117 kann ein flüssiger, methanreicher Strom h und ein gasförmiger, wasserstoffreicher Strom i abgezogen werden. Die Ströme h und i werden nacheinander in den Gegenstromwärmetauschern 114, 113, 112 und 111 erwärmt und einer geeigneten Verwendung, beispielsweise als Heiz- oder Hydriergas, zugeführt.

Aus den Flüssigkeitsabscheidern 115 und 116 sowie der Absorptionskolonne 4 werden flüssige Ströme j, k und l, die überwiegend Kohlenwasserstoffe mit zwei und mehr Kohlenstoffatomen in unterschiedlichen Gehalten und zumindest im Fall des Stroms l auch beträchtliche Mengen Methan enthalten, abgezogen und in eine Destillationssäule 5, den eigentlichen Demethanizer, entspannt.

Die Destillationssäule 5 wird derart betrieben, dass sich an ihrem Kopf ein methanreiches Kopfgas, vorzugsweise im Wesentlichen reines Methan, anreichert. Dieses wird abgezogen, durch einen Kondensationsraum eines in seinem Verdampfungsraum mit einem geeigneten Kältemittelstrom n betriebenen Kopfkondensators 51 geführt und als flüssiger Rücklauf in Form des Stroms m auf die Destillationssäule 5 aufgegeben. Ein Teil des verflüssigten Stroms m kann abgezogen, mittels einer Pumpe 117 auf den Druck der Absorptionskolonne 4 gebracht und in Form des erwähnten Stroms f als Rücklauf verwendet werden. Es sei ausdrücklich betont, dass die Gewinnung der Ströme m und f auch auf andere Weise aus dem Strom m, beispielsweise mittels externer Abscheiderbehälter und/oder unter Verwendung externer Kopfkondensatoren erfolgen kann.

Aus dem Sumpf der Destillationssäule 5 kann ein methanarmer Strom o abgezogen werden, der den überwiegenden Anteil der in die Destillationssäule 5 über die Ströme j, k und l eingespeisten Kohlenwasserstoffe mit zwei und mehr Kohlenstoffatomen enthält. Ein Teil des Stroms o kann in Form des Stroms p in einem Sumpfverdampfer 52 der Destillationssäule 5 verdampft und erneut in diese eingespeist werden, ein weiterer Teil wird als Strom q aus dem Demethanizer 11 entnommen und kann in den Deethanizer 12 überführt werden.

In dem Deethanizer 12 wird der Strom q beispielsweise in einem Wärmetauscher 121 gekühlt und anschließend in eine Destillationssäule 120 mit einem Kopfkondensator 124 und einem Sumpfverdampfer 125 überführt.

Vom Kopf der Destillationssäule 120 kann ein gasförmiger Strom abgezogen werden, der im Wesentlichen aus Kohlenwasserstoffen mit zwei Kohlenstoffatomen besteht und frei von Kohlenwasserstoffen mit drei und mehr Kohlenstoffatomen ist. Ein Teil hiervon wird in dem Kopfkondensator 124 verflüssigt und in Form des Stroms v als Rücklauf auf die Destillationssäule 120 aufgegeben, ein weiterer Teil in Form eines Stroms s aus dem Deethanizer 12 ausgeleitet. Der Strom s wird anschließend typischerweise in eine weitere Destillationssäule, den sogenannten C2-Splitter, überführt, wo die Kohlenwasserstoffe mit zwei Kohlenstoffatomen voneinander getrennt werden. Optionale Zwischenschritte, hier mit Block 6 veranschaulicht, können vorgesehen sein.

Aus dem Sumpf der Destillationssäule 120 kann ein Strom x abgezogen werden, der im Wesentlichen die in dem Strom q enthaltenen Kohlenwasserstoffe mit drei und mehr Kohlenstoffatomen enthalten kann. Ein Teil des Stroms x kann in dem Sumpfverdampfer 125 erwärmt und als aufsteigendes Gas in Form des Stroms y in die Destillationssäule 120 überführt werden, ein anderer Teil wird in Form des Stroms z aus dem Deethanizer 12 ausgeleitet.

Figur 2 veranschaulicht eine Anlage zur Herstellung von Kohlenwasserstoffen gemäß dem Stand der Technik. Die Anlage ist insgesamt mit 300 bezeichnet.

Bezüglich der Schritte bzw. Einrichtungen 1 und 2 sowie der Ströme a bis c sowie der grundsätzlichen Funktionsweise der weiteren dargestellten Einrichtungen sei auf die Erläuterungen zu Figur 1 verwiesen, die hier weiter gelten. Die Trenneinheit 10 der Anlage 300 gemäß Figur 2 implementiert jedoch ein "Deethzanizer First"-Verfahren, d.h. der Produktstrom c wird zunächst dem Deethanizer 12 zugeführt, dem der Demethanizer 11 nachgeschaltet ist. Die sich ergebenden wesentlichen Unterschiede werden nachfolgend erläutert.

In den Deethanizer 12 wird hier ein Strom c eingespeist, der neben den mehrfach erwähnten Kohlenwasserstoffen zusätzlich noch Methan und Wasserstoff enthält. In dem Deethanizer 12 wird der Strom c in einem auch hier mit 121 bezeichneten Wärmetauscher gekühlt und anschließend in eine zweiteilig ausgebildete Absorptionskolonne 122 mit einem Kaminhalsboden überführt, an deren Kopf ein im Wesentlichen Kohlenwasserstoffe mit zwei Kohlenstoffatomen enthaltender, flüssiger Rücklauf r aufgegeben wird.

Vom Kopf der Absorptionskolonne 122 kann auf diese Weise ein gasförmiger Strom abgezogen werden, der Kohlenwasserstoffe mit zwei Kohlenstoffatomen aufweist und frei von Kohlenwasserstoffatomen mit drei und mehr Kohlenstoffatomen ist. Dieser Strom ist, analog zu dem Strom s gemäß Figur 1 bzw. Anlage 200, auch hier mit s bezeichnet. Der Strom s enthält gemäß Figur 2 bzw. Anlage 300 aber auch noch Methan und Wasserstoff. Seine weitere Verwendung ist unten erläutert.

Aus dem Sumpf und vom Zwischenboden der Absorptionskolonne 122 werden Ströme t und u abgezogen, die im Wesentlichen die in dem Strom q enthaltenen Kohlenwasserstoffe mit drei und mehr Kohlenstoffatomen und zudem Kohlenwasserstoffe mit zwei Kohlenstoffatomen enthalten.

Diese Ströme t und u werden in eine Destillationssäule 123 entspannt, die derart betrieben wird, dass sich an ihrem Kopf ein gasförmiges, im Wesentlichen Kohlenwasserstoffe mit zwei Kohlenstoffatomen enthaltendes Kopfprodukt bildet, das abgezogen, durch einen Kondensationsraum eines in seinem Verdampfungsraum mit einem geeigneten Kältemittelstrom w betriebenen Kopfkondensators 124 geführt und als flüssiger Rücklauf auf die Destillationssäule 123 aufgegeben werden kann. Der als Rücklauf auf die Destillationssäule 123 verwendete verflüssigte Strom ist hier, wie der Rücklauf auf die Destillationssäule 120 gemäß Figur 1 bzw. Anlage 200, mit v bezeichnet. Ein Teil des verflüssigten Stroms v kann abgezogen, beispielsweise mittels einer nicht dargestellten Pumpe auf den Druck der Absorptionskolonne 122 gebracht und dort in Form des erwähnten Stroms r als Rücklauf verwendet werden.

Aus dem Sumpf der Destillationssäule 123 kann auch hier ein im Wesentlichen Kohlenwasserstoffe mit drei Kohlenstoffatomen enthaltender Strom x abgezogen werden. Ein Teil des Stroms x kann in Form des Stroms y in einem Sumpfverdampfer 125 der Destillationssäule 5 verdampft und erneut in diese eingespeist werden, ein weiterer Teil wird als Strom z aus dem Deethanizer 12 entnommen und kann beliebigen weiteren Schritten unterworfen werden.

Weil der dem Deethanizer 12 zugeführte Strom c noch Methan und Wasserstoff enthält, gehen diese in den Strom s am Kopf der Absorptionskolonne 122 über. Der Strom s enthält damit gemäß Figur 2 bzw. Anlage 300 auch noch Kohlenwasserstoffe mit zwei und weniger Kohlenstoffatomen und Wasserstoff. Er wird nach beliebigen Zwischenschritten 7 in den Deethanizer 11 überführt und dort wie der Strom c in der Anlage 200 gemäß Figur 1 behandelt.

Aufgrund der vorigen Abtrennung von Kohlenwasserstoffen mit drei und mehr Kohlenstoffatomen in dem Deethanizer 12 ist in der Anlage 300 gemäß Figur 2 jedoch zu vermeiden, dass in dem Demethanizer 11 erneut derartige Kohlenwasserstoffen mit drei und mehr Kohlenstoffatomen eingebracht werden, beispielsweise in Form des Stroms f. Letzterer sollte also möglichst frei von Kohlenwasserstoffen mit drei und mehr Kohlenstoffatomen sein, kann aber ggf. einen gewissen Anteil an Kohlenwasserstoffen mit zwei Kohlenstoffatomen enthalten.

Aufgrund der vorigen Abtrennung von Kohlenwasserstoffen mit drei und mehr Kohlenstoffatomen in dem Deethanizer 12 enthält der Strom q in der Anlage 300 gemäß Figur 2 vorzugsweise keine derartigen Kohlenwasserstoffe mehr; er kann daher wie der Strom s der Anlage 200 gemäß Figur 1 in eine weitere Destillationssäule, den erläuterten C2-Splitter, überführt werden, wo die Kohlenwasserstoffe mit zwei Kohlenstoffatomen voneinander getrennt werden.

Wie aus der Zusammenschau der Figuren 1 und 2 damit ersichtlich, richtet sich die Zusammensetzung der Ströme j, k und l nach der Position der Trennsäule 5 und damit des Demethanizers 11 in der Trennsequenz. Die genannten Ströme können also neben Methan im Wesentlichen nur Kohlenwasserstoffe mit zwei (in "Deethanizer First"-Verfahren), ggf. aber auch Kohlenwasserstoffe mit drei und mehr Kohlenwasserstoffen (in "Demethanizer First"-Verfahren) enthalten. Nicht dargestellt sind weitere Verfahrensvarianten, beispielsweise "Depropanizer First"-Verfahren, bei denen die genannten Ströme neben Methan im Wesentlichen nur Kohlenwasserstoffe mit zwei und drei Kohlenstoffatomen enthalten.

Wie erläutert, kommt es in Anlagen, in denen beispielsweise ethanreiche Kohlenwasserstoffgemische als Einsatzströme a, b eingesetzt werden, bzw. in welchen derartige ethanreiche Kohlenwasserstoffgemische als Frischeinsätze dienen, zu einer deutlichen Reduzierung der Methanmenge im Produktstrom c. Dies bedeutet, dass auch am Kopf der Destillationssäule 5 bzw. an anderer Stelle in der Trennsequenz nur eine vergleichsweise geringe Menge an flüssigem Methan gewonnen werden kann. Hier setzt die vorliegende Erfindung an.

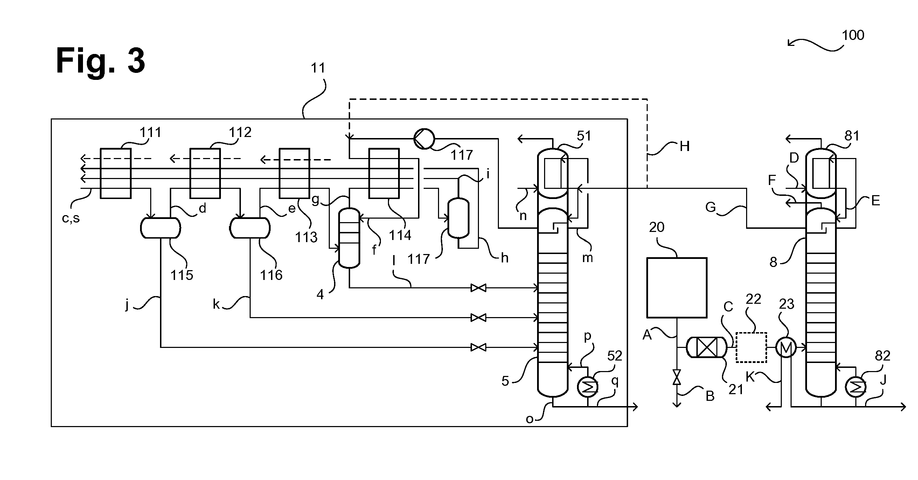

Gemäß einer in Figur 3 veranschaulichten Ausführungsform einer erfindungsgemäßen Anlage 100 ist von den zuvor erläuterten Komponenten nur der Demethanizer 11 gezeigt, die anderen Elemente sind nur der Anschaulichkeit halber in der Zeichnung weggelassen, aber weiterhin vorhanden. Die nachfolgenden Erläuterungen betreffen, bis auf die nachfolgend explizit angesprochenen Unterschiede, sowohl "Demethanizer First"- als auch "Deethanizer First"-Verfahren, die sich im Wesentlichen durch die Zusammensetzung der Ströme c bzw. s unterscheiden.

In der Anlage 100 gemäß einer Ausführungsform der Erfindung ist vorgesehen, den flüssigen Rücklauf m auf die Destillationssäule 5 und/oder den flüssigen Rücklauf f auf die Absorptionskolonne 4 zumindest teilweise aus einem flüssigen, methanreichen Strom G zu bilden. Der Strom G wird wiederum aus einem druckbeaufschlagten, methanhaltigen Gasgemisch, insbesondere Erdgas, erzeugt, das separat zu dem Produktstrom c bereitgestellt wird. Wie erläutert, wird hier mit "separat zu dem Produktstrom c bereitgestellt" ausgedrückt, dass das druckbeaufschlagte, methanhaltige Gasgemisch, aus dem der Strom G und letztlich die flüssigen Rückläufe m bzw. f erzeugt werden, nicht aus dem Produktstrom c oder aus in diesem enthaltenen Komponenten gebildet wird.

In dem in der Figur 3 veranschaulichten Beispiel ist ein Strom eines entsprechenden druckbeaufschlagten, methanhaltigen Gasgemischs mit A bezeichnet. Der Strom A wird beispielsweise über eine Erdgasversorgung 20, insbesondere eine Pipeline, zur Verfügung gestellt. Das druckbeaufschlagte, methanhaltige Gasgemisch des Stroms A liegt beispielsweise in einer Erdgaspipeline mit einem Druck von 40 bis 60 bar vor und eignet sich damit zur Bildung von flüssigem Methan bzw. des Stroms m, der anschließend als flüssiger methanreicher Strom m bzw. f verwendet werden kann.

Ein Teil des druckbeaufschlagten, methanhaltigen Gasgemischs des Stroms A kann beispielsweise als Strom B über ein nicht bezeichnetes Ventil auf einen Druck von weniger als 9 bar entspannt und anschließend als Brenngas verwendet werden. Der Rest wird einer Aufbereitung zugeführt, die beispielsweise auf einem Druck von 10 bis 50 bar, beispielsweise bei 20 bis 45 bar oder 30 bis 40 bar, arbeitet. Der Druck liegt jedenfalls unterhalb des kritischen Drucks von Methan.

Entsprechende Gasgemische aus Pipelines, wie beispielsweise das druckbeaufschlagte, methanhaltige Gasgemisch des Stroms A, enthalten typischerweise noch Spurenverunreinigungen wie Arsin, Phosphin, Schwefelverbindungen, Kohlendioxid und Quecksilber. Derartige Verunreinigungen können in einer adsorptiven Reinigungseinrichtung 21 entfernt werden, in der auch beispielsweise die unten erläuterten Ströme F und J zur Regenerierung eingesetzt werden können.

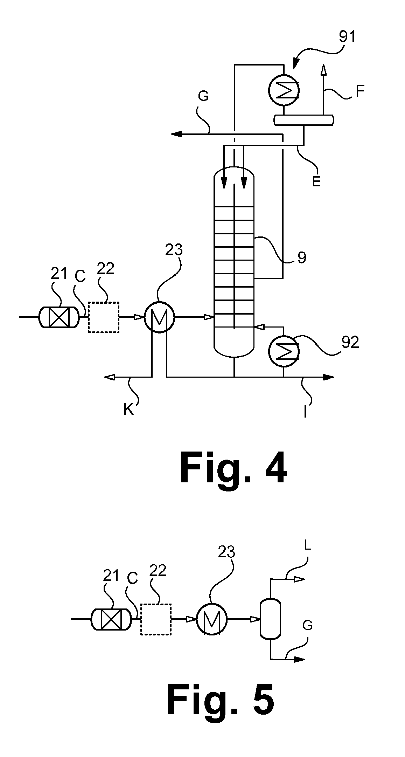

Ein entsprechend aufgereinigter Strom C wird optional beliebigen weiteren Aufbereitungsschritten, hier mit 22 bezeichnet, unterworfen, anschließend in einem Wärmetauscher 23 abgekühlt und schließlich in geeigneter Höhe in eine Destillationssäule 8 überführt. Die Destillationssäule 8 dient zur Gewinnung eines methanreichen Kopfstroms aus dem methanhaltigen, druckbeaufschlagten Gasgemisch der Ströme A bzw. C. Liegt der Strom C bereits in ausreichender Reinheit, d.h. insbesondere mit ausreichendem Methangehalt vor, ist auch eine reine Verflüssigung ohne den Einsatz einer Destillationssäule 8 möglich, wie unten unter Bezugnahme auf Figur 5 erläutert. Wie erwähnt, richten sich die zulässigen Gehalte höherer Kohlenwasserstoffe in den Strömen m bzw. f insbesondere nach der Position der Destillationssäule 5 bzw. der Absorptionskolonne 4 in der Trennsequenz ("Demethanizer First"- oder "Deethanizer First"-Verfahren).

In einem Kopfkondensator 81 der Destillationssäule 8 kann unter Verwendung eines geeigneten Kältemittels in Form des Stroms D ein gasförmiger methanreicher Kopfstrom der Destillationssäule 8 zu einem Strom E verflüssigt und zumindest zum Teil am Kopf der Destillationssäule 8 erneut aufgegeben werden. Ein in dem Kopfkondensator 81 der Destillationssäule 8 nicht verflüssigter Anteil kann als Strom F abgezogen und beispielsweise als Brenngas verwendet werden. Der mehrfach erläuterte Strom G, der im Wesentlichen aus flüssigem Methan besteht, kann vom Kopf der Destillationssäule 8 über eine geeignete Flüssigkeitsentnahmeeinrichtung entnommen werden. Es sei nochmals ausdrücklich betont, dass die Erfindung nicht auf das dargestellte konkrete Beispiel eines Kopfkondensators beschränkt ist. Die Ströme F und G können auch auf andere Weise, beispielsweise in einem externen Abscheider, gewonnen werden.

Im Sumpf der Destillationssäule 8 scheidet sich eine flüssige Fraktion ab, die überwiegend aus Kohlenwasserstoffen mit zwei und mehr Kohlenstoffatomen bestehen kann. Es ist jedoch auch möglich, die Destillationssäule 8 derart zu betreiben, dass sich in ihrem Sumpf ein Gasgemisch abscheidet, das auch weitere, abzutrennende Komponenten enthält. Auch kann im Sumpf der Destillationssäule 8 noch eine beträchtliche Menge an Methan enthalten sein. Wichtig ist, dass am Kopf der Destillationssäule 8 eine Fraktion gebildet wird, die die oben erläuterte Verwendung zulässt und nur geeignete Komponenten aufweist, beispielsweise neben Methan nur Kohlenwasserstoffe mit zwei Kohlenstoffatomen und diese in begrenzter Menge für die "Deethanizer First"-Verfahren bzw. auch Kohlenwasserstoffe mit drei und mehr Kohlenstoffatomen in begrenzter Menge für "Demethanizer First"-Verfahren.

Die Destillationssäule 8 kann ferner bei unterschiedlichen Betriebsdrücken betrieben werden, die ebenfalls davon abhängen können, für welche Zwecke der Strom G verwendet werden soll. Für den Einsatz in einer Absorptionskolonne 4 eines C2-Absorbers sind beispielsweise Drücke von 28 bis 36 bar erforderlich, wozu in herkömmlichen Verfahren die Pumpe 117 eingesetzt wird. Wird die Destillationssäule 8 bei entsprechend hohem Druck betrieben, kann, wie hier mit Strom H veranschaulicht, ein entsprechender Strom auch stromab einer solchen Pumpe 117 eingespeist werden. Letztere kann kleiner dimensioniert und kostengünstiger ausgelegt werden. Zur Verwendung als Rücklauf auf die Destillationssäule 5 selbst sind hingegen ggf. geringere Drücke von beispielsweise 13 bis 36 bar erforderlich. Eine weiterhin mögliche Methanrückführung vor oder in die Rohgasverdichtung (siehe Erläuterungen eingangs) ermöglicht beispielsweise den zuvor genannten geringen Druck.

Methan der Ströme G bzw. H kann auch, wie hier nicht gesondert veranschaulicht, zusätzlich beispielsweise in dem Wärmetauscher 113 erwärmt werden.

Ein aus dem Sumpf der Destillationssäule 8 abgezogener Strom J, der nicht in dem Sumpfverdampfer 92 der Destillationssäule 8 verdampft wird, kann ebenfalls als Brenngas verwendet werden, so dass seine Zusammensetzung im Gegensatz zu dem Strom G weniger kritisch ist. Wie mit Strom K veranschaulicht, kann mit einem aus dem Sumpf der Destillationssäule 8 abgezogenen Strom auch der Wärmetauscher 23 betrieben werden. Auf einen Sumpfverdampfer 82 kann ggf. auch verzichtet werden.

Anstelle der einfachen Destillationssäule 8 kann auch eine Trennwandkolonne eingesetzt werden. Die Verwendung einer Trennwandkolonne trägt der Tatsache Rechnung, dass beispielsweise Erdgas, das in Form des druckbeaufschlagten, methanhaltigen Gasgemischs des Stroms A bereitgestellt wird, typischerweise beträchtliche Mengen an Stickstoff enthält. Um zu vermeiden, dass entsprechender Stickstoff in den Strom G und damit in den Strom m bzw. f übergeht, wird in der Trennwandkolonne eine Abreicherung von Stickstoff erzielt. Entsprechender Stickstoff könnte anderenfalls Produkte der Anlage 100 verunreinigen.

In Figur 4 ist ein Ausschnitt einer erfindungsgemäßen Anlage gezeigt, in der eine entsprechende Trennwandkolonne mit 9 bezeichnet ist. Die Einbindung ergibt sich aus der Bezeichnung der jeweiligen Ströme. Die Trennwandkolonne 9 umfasst im dargestellten Beispiel einen Kopfkondensator 91 und einen Sumpfverdampfer 92. Der noch stickstoffhaltige Gasstrom C wird nach der Abkühlung in dem Wärmetauscher 23 in einen hier links dargestellten Bereich der Trennwandkolonne 9 eingespeist. In einem rechts dargestellten Bereich der Trennwandkolonne 9 kann stickstoffabgereichertes Methan bzw. ein Gasgemisch geeigneter Zusammensetzung abgezogen und als der Strom G verwendet werden.

Eine besonders einfache Variante ist in Figur 5 veranschaulicht. Hier wird der Strom G lediglich durch Verflüssigung eines entsprechend aufgereinigten Stroms A bzw. C erhalten. Diese Variante eignet sich für Fälle, in denen das Gasgemisch des Stroms C bereits eine spezifikationsgerechte Zusammensetzung aufweist, insbesondere für "Demethanizer First"-Verfahren, in denen Kohlenwasserstoffe mit drei und mehr Kohlenstoffatomen im Strom G toleriert werden können. Ist der Strom A bereits frei von anderen störenden Verunreinigungen, kann auch auf die Reinigungseinrichtung 21 verzichtet werden.

## Patentansprüche

1. Verfahren zur Herstellung von Kohlenwasserstoffen, bei dem zumindest ein in einer Reaktionseinheit (1) erzeugter kohlenwasserstoffhaltiger Produktstrom (c) oder zumindest ein aus dem Produktstrom (c) gebildeter Strom (d, e, g, j, k, l) in zumindest einer Trenneinheit (10) unter Verwendung zumindest eines flüssigen, methanreichen Stroms (f, m) kryogen behandelt wird, **dadurch gekennzeichnet, dass** der zumindest eine methanreiche Strom (f, m) zumindest teilweise unter Verwendung eines druckbeaufschlagten, methanhaltigen Gasgemischs (A) erzeugt wird, das separat zu dem Produktstrom (c) bereitgestellt wird.

2. Verfahren nach Anspruch 1, bei dem die kryogene Behandlung ein Abkühlen in zumindest einem mit dem zumindest einen methanreichen Strom (f) als Kältemittel betriebenen Wärmetauscher (114) umfasst.

3. Verfahren nach Anspruch 1, bei dem die kryogene Behandlung ein Trennen in zumindest einer kryogenen Trenneinrichtung (4, 5) umfasst, in der der zumindest eine methanreiche Strom (f, m) als Rücklauf aufgegeben wird.

4. Verfahren nach Anspruch 3, bei dem zumindest eine Absorptionskolonne (4) und/oder zumindest eine Destillationssäule (5) als die zumindest eine kryogene Trenneinrichtung verwendet wird.

5. Verfahren nach einem der vorstehenden Ansprüche, bei dem die Reaktionseinheit als Dampfspalteinheit (1) mit wenigstens einem Spaltofen ausgebildet ist.

6. Verfahren nach einem der vorstehenden Ansprüche, bei dem Erdgas als das druckbeaufschlagte, methanhaltige Gasgemisch (A) verwendet wird.

7. Verfahren nach einem der vorstehenden Ansprüche, bei dem der flüssige, methanreiche Strom (f, m) zumindest teilweise aus einem flüssigen Strom (M) erzeugt wird, der unter Verwendung eines Destillationsverfahrens aus dem druckbeaufschlagten, methanhaltigen Gasgemisch (A) gebildet wird.

8. Verfahren nach Anspruch 7, bei dem das druckbeaufschlagte, methanhaltige Gasgemisch (A) vor der Durchführung des Destillationsverfahrens zumindest teilweise unter Druck von Verunreinigungen befreit wird.

9. Verfahren nach Anspruch 8, bei dem Arsin, Phosphin, Schwefelverbindungen, Kohlendioxid und/oder Quecksilber zumindest teilweise aus dem druckbeaufschlagten, methanhaltigen Gasgemisch (A) entfernt werden.

10. Verfahren nach einem der Ansprüche 7 bis 9, bei dem das druckbeaufschlagte, methanhaltige Gasgemisch (A) in dem Destillationsverfahren an Stickstoff, Wasserstoff und/oder Helium abgereichert wird.

11. Verfahren nach Anspruch 10, bei dem zur Abreicherung des druckbeaufschlagten, methanhaltigen Gasgemischs (A) an Stickstoff, Wasserstoff und/oder Helium in dem Destillationsverfahren eine Trennwandkolonne (6) verwendet wird.

12. Anlage (100, 200, 300), die sämtliche, zur Durchführung eines Verfahrens nach einem der vorstehenden Ansprüche eingerichtete Mittel aufweist und zur Durchführung eines entsprechenden Verfahrens ausgebildet ist.
